# EUROPÄISCHE PATENTSCHRIFT

(11) **EP 1 207 335 B1**
(45) Veröffentlichungstag und Bekanntmachung des Hinweises auf die Patenterteilung: **14.06.2006**
(21) Anmeldenummer: 01125607.0
(22) Anmeldetag: 26.10.2001
(51) Int. Cl.: F16M 11/04

(54) **Stativ**
Stand
Support

(30) Priorität: 12.11.2000 DE 20019107 U
(43) Veröffentlichungstag der Anmeldung: 22.05.2002
(73) Patentinhaber: Leica Microsystems AG, 9435 Heerbrugg (CH)
(72) Erfinder: Metelski, Andrzej, 8590 Romanshorn (CH)
(74) Vertreter: Reichert, Werner Franz

(56) Entgegenhaltungen:
- EP-A- 0 342 560
- DD-A- 221 571
- DE-A- 3 739 080
- DE-A- 19 742 050
- DE-U- 20 019 105
- DE-U- 20 019 106
- DE-U- 20 019 107
- DE-U- 20 019 109
- US-A- 3 820 752
- US-A- 4 515 333
- US-A- 4 544 121
- US-A- 5 397 323
- US-A- 6 070 839
- US-A- 6 129 319

## Beschreibung

Die Erfindung betrifft ein Stativ, insbesondere für Operationsmikroskope, gemäss Oberbegriff des Anspruches 1.

Operationsmikroskope müssen über einen vorgegebenen Bereich leicht schwenkbar sein und sollten eine eingestellte Position beibehalten. Aus diesem Grund sind bei bekannten Stativen Ausgleichsgewichte oder Kompensationsfedern vorgesehen, die das Gewicht des Mikroskops und seiner Zusatzeinrichtungen kompensieren. Am häufigsten werden die Ausgleichsgewichte balkenwaagenartig angeordnet.

Besondere Ausbildungsformen solcher Balkenwaagen-Anordnungen sind beispielsweise der Aufbau "OHS⁽™⁾" der Anmelderin, bei dem über Parallelogrammträger Ausgleichsgewichte von oben in den unteren Bereich des Stativs verlegt sind, sodass der Gesamtschwerpunkt des Stativs im unteren Drittel des Stativaufbaus liegt. Der Prinzipaufbau des OHS^{(™)} ist in der internationalen Patentanmeldung WO 97/13997 der Anmelderin symbolisch dargestellt. Bei diesem Aufbau bilden Stangen die Verbindung zwischen Tragarm und Ausgleichsgewicht.

Die Erfindung beschäftigt sich insbesondere mit der Frage nach erhöhter Betriebssicherheit bei Stativen. Die nachfolgenden Angaben aus dem Stand der Technik zeigen, dass es bisher für seilgestützte, aber auch für andere Mikroskopstative noch keine optimalen Sicherheitsvorkehrungen im Falle von Materialbrüchen gibt.

In der DE 197 42050 A1 wird Bezug genommen auf einen Aufsatz "Gewichtsausgleich an feinmechanischen Geräten" von H. Hilpert in Heft 2/1965 der Zeitschrift Feingerätetechnik, 14. Jahrgang.

In diesem Artikel aus dem Jahr 1965 wird auf verschiedene gewichtskompensatorische Massnahmen in der Feingerätetechnik eingegangen, die in erster Linie nicht durch ein Gegengewicht, sondern durch federkompensatorische Massnahmen (wie vergleichsweise auch bei der Scherenarmkonstruktion des MS 1 der Anmelderin) erzielt werden. Sicherheitsaspekte spielen dabei keine Rolle.

Die DD 221571 A1 (1985) zeigt einen Stativaufbau mit einem Hebelarm, an dessen distalem Ende sich das Operationsmikroskop befindet. Das Gewicht des Mikroskops wird durch eine Feder kompensiert, die über einen Seilzug mit dem Hebelarm verbunden ist. Die Grundjustierung dieses Operationsmikroskops erfolgt mittels einer Gewindespindel, mit der das gehäuseseitige Ende der Feder weiter vom Hebelarm weggezogen oder näher zu ihm hingeführt wird. Gewichtsänderungen am Mikroskop werden dadurch kompensiert, dass der Anlenkpunkt des Seilzugs relativ zum Hebelarm ebenfalls über eine Spindel verstellt wird.

Um in allen möglichen Winkellagen ein gleichmässiges Gegenmoment zu erzielen, ist es erforderlich, dass sich der erwähnte Angriffspunkt des Seilzugs auf einer Verbindungslinie zwischen der Drehachse des Hebelarms und dem Masseschwerpunkt des Mikroskops befindet. Dies wird durch das Betätigen einer Verstelleinrichtung in Form einer Schnecke erreicht, die eine mit dem Hebelarm verbundene Scheibe um die Drehachse des Hebelarms dreht.

Die DE 3739080 A1 (1989) gibt ebenso eine Federvorrichtung zum Gewichtsausgleich für Stative an, bei der Seilzüge mit Federn kombiniert zu einem Gewichtsausgleich führen sollen. Dabei geht es um die Kraftunterstützung einer Verstellbewegung, die eine Bedienperson an einem Handgriff ausübt und nicht darum, eine Last in einem "schwebenden Zustand" zu halten, wie dies bei Operationsmikroskopen gewünscht ist. Im Falle eines Bruchs der Feder oder des Seiles entfällt die Gewichtskompensation und die Last kann plötzlich absinken.

Demgegenüber ist in der US 5397323 (1992) ein Operationsroboter mit Parallelogrammträgern vorgestellt, bei dem unter anderem das Gewicht des Instruments über einen Seilzug mittels Gegengewicht gewichtskompensiert gehalten wird. Der Seilzug ist dabei geschlossen aufgebaut, d.h. über eine obere und untere Umlenkrolle wird je ein Seil vom Instrument bis zum Gegengewicht geführt (Fig.3 der US 5397323).

Die DE 19742050 A1 (1999) offenbart einen Stativaufbau mit einem schwenkbaren Parallelogrammträger, der über einen Seilzug und eine Gewichtsausgleichsfeder so gewichtskompensiert ist, dass die zusätzlich vorhandenen Ausgleichsgewichte, die nach dem oben erwähnten Waagenprinzip wirken, besonders klein ausgebildet sein können. Bei diesem Aufbau wird der Seilzug in besonderer Form geführt, um den durch den endlichen Umlenkradius bedingten Gewichtsausgleichsfehler in einem weiten Schwenkbereich des Schwenkarms zu minimieren. Der Gewichtsausgleichsfehler wird durch diese Massnahme jedoch nicht eliminiert, sodass bei bestimmten Schwenkpositionen nach wie vor das Verstellen der Ausgleichsgewichte erforderlich ist.

Die US 6070839 (2000) offenbart einen weiteren Aufbau mit einem Schwenkarm und einer Seilzug-Feder-Konstruktion, die einen reinen Gewichtsausgleich ermöglicht. Im Falle von Gewichtsänderungen wird der Anlenkpunkt des Seilzugs, vergleichbar dem Aufbau in der erwähnten DD 221571, über eine Spindel verschoben. Ein allfälliger Materialbruch wird in dieser Schrift nicht behandelt.

Die US 5253832 (1999) beschreibt ein Stativ mit einem Seilzug und einer zentral angeordneten Zugfeder für den Gewichtsausgleich. Dieser Aufbau bietet keine einfache Verstellmöglichkeit für geänderte Lasten. Das Seil übernimmt die volle Last des Tragarms und des Mikroskops.

Bei einem Aufbau gem. der EP-A-866260 wird zur Übertragung von Ausgleichsgewichten ein Zahnriemen eingesetzt, der mittels Bremse eingebremst gehalten werden kann. Falls es beim Zahnriemen zu einem Bruch kommt, so kann dies zu einem Absturz des Mikroskops auf den darunter liegenden Patienten am Operationstisch führen.

Sämtliche der bisher bekannten und oben angegebenen Stativaufbauten mit Seilzügen oder Zahnriemen weisen die gleiche Problematik auf: Im Falle eines Bruchs des Seiles oder einer Zugfeder kommt es zu einer erheblichen Funktionsstörung des Gerätes, die in der Regel zu einem plötzlichen Absinken der Last, im Fall eines Operationsmikroskops zum rapiden, unzulässigen Absinken des Mikroskops führen kann.

Insbesondere während einer Operation könnte ein solcher Bruch für den Patienten katastrophale Folgen haben. Die naheliegende Lösung wäre, das zu verwendende Seil entsprechend stark zu dimensionieren, sodass ein Bruch praktisch unmöglich ist. Mit erhöhter Materialstärke werden die Seilzüge jedoch unbeweglicher.

Bei der Dimensionierungsfrage müsste auch berücksichtigt werden, dass in extremen Situationen die Last bzw. das Gewicht am Lastangriffspunkt aussergewöhnlich hoch ansteigen kann. Dies ist beispielsweise der Fall, wenn sich ein Operateur - ggf. vielleicht wegen Übelkeit - kurzfristig am Mikroskop abstützt.

Der Erfindung liegt nun die Aufgabe zugrunde, einen Sicherheitsmechanismus zu finden, der im Falle eines Seilbruchs nicht zum Absinken der Last führt. Die Erfindung soll dabei nicht allein auf die Verwendung bei einem Stativ für Operationsmikroskope eingeschränkt sein, sondern vielmehr auch beliebige Formen von Stativen umfassen, bei denen mittels Seilzügen Kraft- und/oder Gewichtsausgleiche vorgenommen werden.

Gelöst wird diese Aufgabe durch das Zurverfügungstellen wenigstens eines zweiten Seiles, wobei das zweite Seil gleichberechtigt zum ersten Seil aufgebaut ist und parallel die gleichen Funktionen ausübt, solange das erste Seil intakt ist. Im Sicherungsfall (d.h. Bruch eines der beiden Seile) übernimmt es alleine die Arbeitsseilfunktion.

Alternativ ist dem wenigstens einen Arbeitsseil mindestens ein zweites Seil als Sicherheitsseil zur Seite gestellt, das im Betriebszustand spannungslos ist und nur im Sicherungszustand die Funktionen des Arbeitsseils übernimmt. Dies führt erfindungsgemäss dazu, das trotz der Anwendung von zwei Seilzügen der Arbeitswiderstand im wesentlichen nicht höher ist als bei Konstruktionen mit nur einem Seil, da das Sicherungsseil praktisch reibungs- und daher widerstandslos mitläuft.

Im Sinne der Erfindung umfasst der Begriff "Seil" alle jene Konstruktionselemente, die seil-, riemen- oder kettenähnlich aufgebaut sind und der Lastübertragung dienen.

Im Fall eines Bruchs des Arbeitsseils kommt es somit nur zu einer minimalen Bewegung der Last (bis zum Spannen des Sicherungsseils), die somit in der Regel keine Gefährdung darstellt. Bei gleichberechtigten Arbeitsseilen ohne eigentliches Sicherungsseil erfolgt gegebenenfalls ebenso eine Anpassung im Sicherungsfall über ein wippenähnliches Bauteil - vorzugsweise mit einer Wegbegrenzung zur Begrenzung der Wippbewegung.

Gemäss einer besonderen Ausgestaltung der Erfindung ist ein weiterer Sicherungsmechanismus vorgesehen, der verhindern soll, dass das Stativ über einen unbeschränkten Zeitraum weiterverwendet wird, obwohl ein Arbeitsseil gebrochen ist.

Ohne diesen weiteren Sicherungsmechanismus würde die Bedienperson praktisch keinen Unterschied zwischen dem Arbeiten mit dem Arbeitsseil und dem Arbeiten mit dem Sicherungsseil bemerken. Im Falle des gebrochenen Arbeitsseils läuft, gemäss der grundsätzlichen Ausbildung der Erfindung, die Vorrichtung unverändert wie zuvor mit intaktem Arbeitsseil, aber lockerem Sicherungsseil. Die weitere Sicherungsvorrichtung sieht vor, dass, sobald das Sicherungsseil aktiv ist, eine Bremse automatisch in Funktion tritt, die den Arbeitswiderstand am Gerät, d.h. den Widerstand, den der Anwender beim Verschwenken der Last verspürt, deutlich erhöht.

Dieser hohe Arbeitswiderstand soll dem Anwender zeigen, dass das Gerät dringend einem Service unterzogen werden muss. Im Zuge des Services kann dann das gebrochene Arbeitsseil ersetzt und der ursprüngliche Zustand wiederhergestellt werden. Der Arbeitswiderstand ist jedoch nicht so gross, dass eine Operation infolge Nichtbedienbarkeit des Stativs unterbrochen oder abgebrochen werden müsste.

Bei einer besonderen Ausgestaltung der Erfindung wird der zweite Sicherungsmechanismus selbsttätig aktiviert, indem das Sicherungsseil, das ursprünglich nicht belastet ist. Sobald es auf Zug belastet ist, wird das Sicherungsseil in eine keilförmige Nut in einer Umlenkrolle hineingezwängt, in der es einem hohen Reibungswiderstand unterliegt. Dies im Unterschied zu einer üblichen hohlkellenförmig geformten Laufnut für das Arbeitsseil, die dem Seillauf möglichst wenig Reibungswiderstand entgegensetzt. Die keilförmige Nut kann durch eine Schutzfolie aus Kunststoff, Metall o.dgl. gegen ein unbeabsichtigtes Einfressen des Seiles in die Keilnut geschützt sein, die erst dann bricht und den Weg zum Einfressen in die Keilnut freigibt, wenn auf das Sicherungsseil eine Zugkraft von bestimmter Grösse ausgeübt wird.

Eine alternative Variante zum erwähnten Aufbau mit zusätzlicher Sicherung ist ein Klemmelement, das in einer solchen Relativposition zum Sicherungsseil angeordnet ist, dass dieses im unbelasteten Zustand lose durch das Klemmelement hindurchgeht, während es im belasteten Zustand (in gespanntem Zustand) in das Klemmelement eindringt und dort einer erhöhten Reibung unterliegt.

Das Sicherungsseil wirkt somit mit einem Bremskörper zusammen, der - wenn das Sicherungsseil im Bruchfall des Arbeitsseils die Zugfunktion übernommen hat - das Sicherungsseil oder einen mit ihm verbundenen Bauteil bremst, sodass nur noch ein Notbetrieb des Stativs möglich ist. Der Bremskörper kann eine eigene Seilbackenbremse sein (wie bspw. bei einem Segelschiff), es kann aber auch eine Klinkenbremse sein (wie bei einer Sicherheitsgurtautomatik) oder eine Laufrolle mit besonders tiefer V-Nut.

Dieser Aufbau kann durch eine Weiterbildung noch dahingehend verbessert werden, dass das Sicherungsseil durch einen Spanner in Löserichtung gedrückt gehalten ist, wobei der Spanner mit einer relativ geringen Federkraft federbeaufschlagt ist, die im Falle des Seilbruchs des Arbeitsseils durch die Spannung im Sicherungsseil leicht überwunden werden kann.

In einer weiteren Ausgestaltung der Erfindung ist es vorgesehen, das Sicherungsseil mittels einer Andrückrolle in Löserichtung gedrückt zu halten .

Es kann jedoch auch vorgesehen sein, wenigstens eine der Umlenkrollen und/oder den Tragarm mittels einer Bremse bremsbar auszubilden.

In einer weiteren Ausgestaltung der Erfindung sind die Arbeits- und/oder Sicherungsseile oder wenigstens eine Umlenkrolle und/oder wenigstens eine Bremse mit einer elektrischen und/oder elektromotorischen und/oder elektromagnetischen und/oder hydraulischen und/oder pneumatischen und/oder computerunterstützten Steuerung kombiniert.

Die vorliegende Erfindung wird mit Vorzug bei einem Stativaufbau gem. der am gleichen Tag angemeldeten Patentanmeldungen DE 200 19 109, DE 200 19 105 und DE 200 19 106 zum Einsatz gelangen. Sie ist jedoch auf solche Aufbauten nicht eingeschränkt.

Unter Trägern im Sinne der Patentansprüche sind sowohl einzelne Tragarme als auch Parallelogrammträger oder ähnliche Konstruktionen zu verstehen.

Im Folgenden ist die Erfindung anhand von Zeichnungen beispielhaft erläutert. Dabei zeigen:
- Fig.1 -: Das Prinzip eines Stativs mit Tragarm, Gleitschuh, Seilzug und parallel geführtem Sicherungs-Seilzug;
- Fig.1a -: eine Variante mit Federzug;
- Fig.2 -: den normalen Arbeitszustand des Aufbaus gem. Fig.1, in perspektivischer Ansicht;
- Fig.3 -: den gesicherten Zustand des Aufbaues gemäss Fig.1, mit gerissenem Arbeitsseil;
- Fig.4 -: eine Umlenkrolle mit den zwei Seilen;
- Fig.4a -: ein Detail aus Fig.4;
- Fig.4b: eine Variante zu Fig.4a;
- Fig.5 und 5a -: eine mehrteilige Variante der Umlenkrolle
- Fig.6 und 6a -: einen alternativen Aufbau mit einer Keilbremse, die nicht mit den Umlenkrollen verbunden ist;
- Fig.7 und 7a -: einen vergleichbaren Aufbau zu Fig.6 und 6a mit an einem vertikal verlaufenden Seilzug angeordneten Klemmelement
- Fig.8 -: eine weitere Variante zur zusätzlichen Erhöhung der Sicherheit bei einem Aufbau, bei welchem der Ausgleich mit mehreren Seilen vorgenommen wird;
- Fig.9 und 9a -: eine Seilbremse in Betriebs- bzw. Sicherungsstellung;
- Fig.10 -: eine Seilbremse für das Sicherungsseil; in perspektivischer Ansicht;
- Fig.11 -: einen Längsschnitt durch einen Tragarm mit Seil und Flaschenzug;

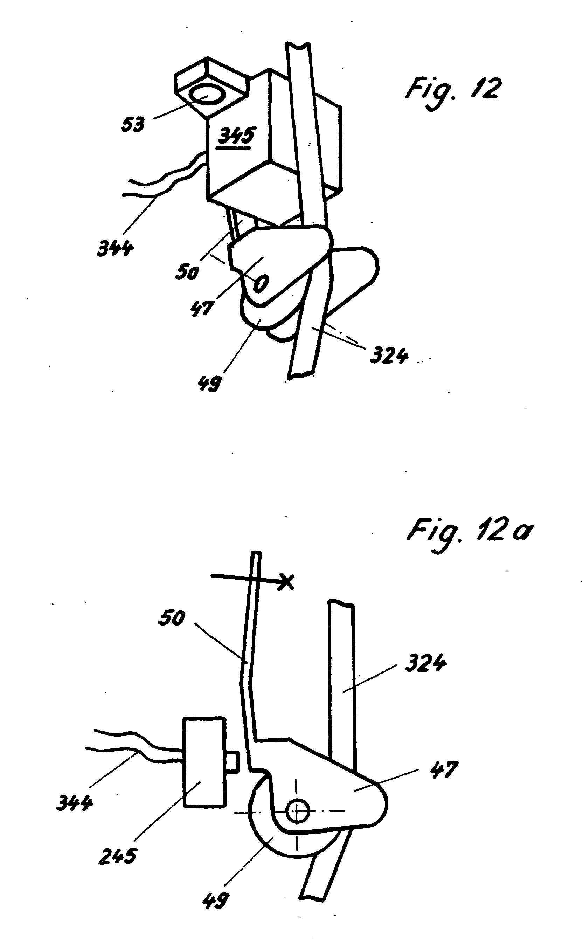

- Fig.12: und 12a - eine Variante mit einem elektrischen Endschalter für die Signalabgabe und/oder Steuerung der Bremse im Sicherungsfall und
- Fig.13: und 13a - eine Variante zum Aufbau nach Fig.8, bei der lediglich zwei Arbeitsseile 24d vorgesehen sind, die grundsätzlich immer gespannt sind.

Die Figuren werden übergreifend beschrieben, gleiche Bezugszeichen bedeuten gleiche Bauteile, Bezugszeichen mit gleichen Nummern, jedoch unterschiedlichen Indizes bedeuten geringfügig unterschiedliche Bauteile mit gleichen Aufgaben bzw. ähnlichen Wirkungen

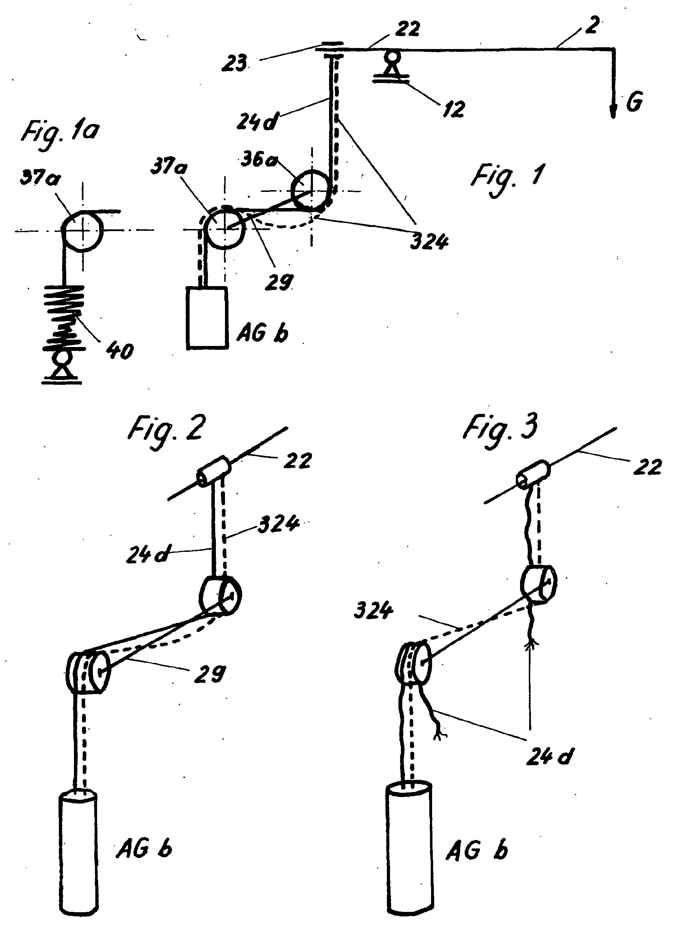

In Fig.1 ist ein Stativ mit Tragarm 2, Ausgleichsarm 22 und darauf verschließbarem Gleitschuh 23 dargestellt, der in den Fig.2 und Fig.3 weggelassen wurde. In der Regel wird ein erfindungsgemässer Aufbau mit einem Gleitschuh aufgebaut sein, jedoch liegen im Rahmen der Erfindung auch Varianten mit fix montierten Seilzügen 24 bzw. Sicherungsseilen 324.

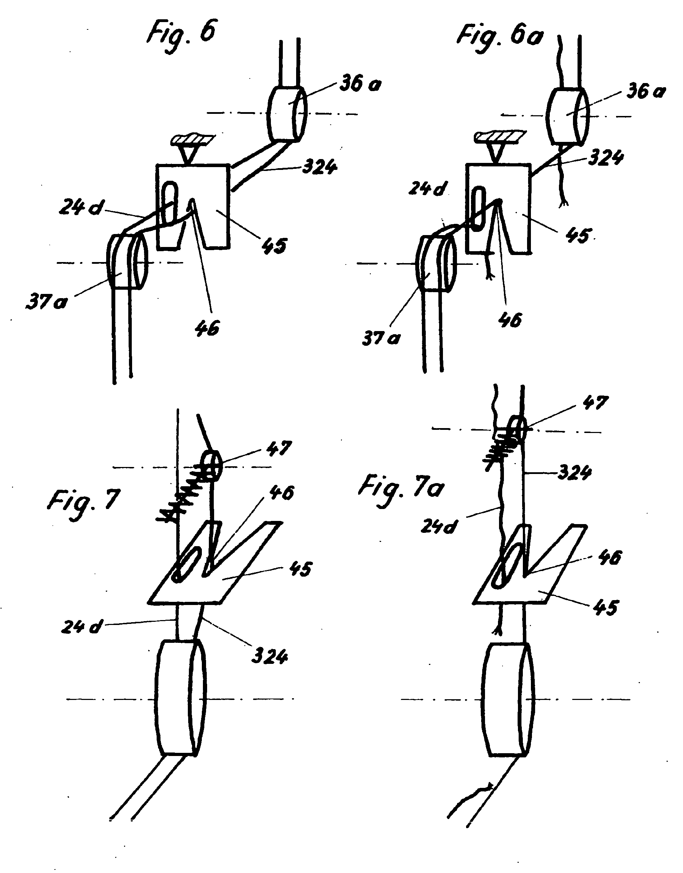

Alternativ ist auch ein Aufbau bevorzugt, bei dem der Gleitschuh 23 durch ein Übertragungselement - insbesondere eine Rolle (30) - ersetzt wird, die einen Bügel (32) stützt, an dem die Seile 24 bzw. 324 befestigt sind. Die beiden Seile sind über Umlenkrollen 36a und 37a geführt, die im Unterschied zu den dort mit 36 und 37 bezeichneten Umlenkrollen gem. Fig.7 der erwähnten Patentanmeldung DE 200 19 105 doppelspurig geführt sind, sodass sowohl dem Arbeitsseil 24d, als auch dem Sicherungsseil 324 eine Spurrille zur Verfügung steht.

Am unteren Ende der beiden Seilzüge 24d und 324 sind diese mit einem Ausgleichsgewicht AGb verbunden, das mit konstanter Kraft F über den Seilzug 24d den Ausgleichsarm 22 in Ausgleichsrichtung mit einem Moment, das durch die Last G und den Tragarm 2 erzeugt wird, belastet.

Alternativ zum Gewicht AGb könnte im Rahmen der Erfindung auch eine Zugfeder 40 o.dgl. vorgesehen sein, wie dies symbolisch in Fig. 1a angedeutet ist. Zwischen den beiden Umlenkrollen 36a und 37a erkennt man das gespannte Arbeitsseil 24d und das lose durchhängende Sicherungsseil 324. Das Sicherungsseil 324 ist gestrichelt gezeichnet, um es vom Arbeitsseil 24d optisch abzuheben.

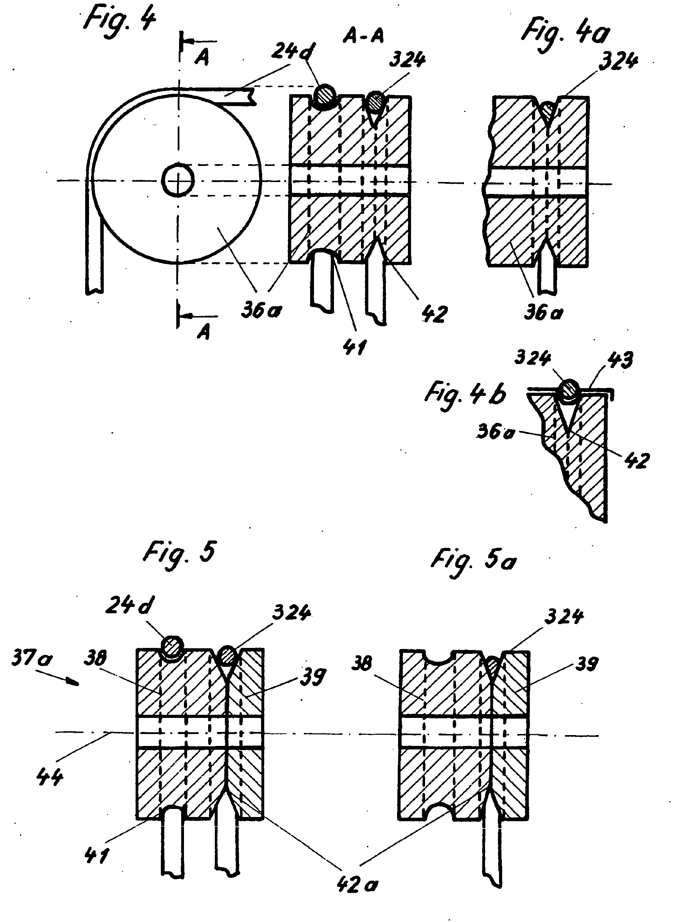

Beim Aufbau nach Fig.4 erkennt man, dass die Nut 41 für das Arbeitsseil 24d dem Querschnitt des Arbeitsseils angepasst ist, während die Klemmnut 42 im Querschnitt keilförmig ausgebildet ist und das Sicherungsseil 324 - sofern unter Spannung - unter hoher Reibung im inneren Bereich der Klemmnut 42 festgefressen wird, wie beispielsweise in Fig.4a angedeutet.

Eine Variante zu einer einfachen Keilnut als Klemmnut 42 stellt der Aufbau gem. Fig.4b dar, bei dem ein Sicherungsmantel 43 über die Klemmnut 42 gelegt ist und das Sicherungsseil 324 trägt, solange es kraftlos auf der Rolle 36a anliegt. Sobald jedoch - nach Bruch des Arbeitsseils 24d - das Sicherungsseil 324 die Funktion des Arbeitsseils übernimmt, wird der leicht brüchige oder elastische Sicherungsmantel 43 den Weg für das Festfressen des Sicherungsseiles 324 in die Klemmnut 42 freigeben.

Die Umlenkrolle 37b ist beim Ausführungsbeispiel nach Fig.5 zweigeteilt in einen drehbaren Teil 38 und einen starren Teil 39, nebeneinander auf einer Achse 44 befestigt sind. Der starre Teil 39 könnte auch drehbar, jedoch stark gebremst, auf der Achse 44 montiert sein. Im Normalbetrieb rotiert die Rolle 38 somit ungehindert und das Arbeitsseil 24d überträgt die Zugkräfte. Im Sicherungsfall, d.h. Wenn das Arbeitsseil 24d reisst, frisst sich das Sicherungsseil 324 zwischen die frei drehbare Rolle 38 und die gebremste oder starre Rolle 39. Dies führt, wie in Fig.5a ersichtlich, zum Bremseffekt im keilförmigen Spalt zwischen den Rollen 38 und 39. Die Bremswirkung auf die bremsbare Rolle 39 kann z.B. auf drei unterschiedliche Arten realisiert sein:
a) Der Teil 39 kann am Gehäuse festgelegt sein.
b) Der Teil 39 kann gegenüber dem Teil 38 axial verschoben und dadurch in oder gegen eine Bremse gedrückt werden. Die axiale Verschiebung entsteht typisch durch das Einfressen des Sicherungsseiles 324 im Sicherungsfall, da es über die Keilfläche eine Spreizwirkung erzeugt. Die Teile 38 und 39 können zueinander federbelastet sein.
c) Dem Teil 39 ist eine von aussen angreifende Bremse zugeordnet, die im Bedarfsfall - ggf. sensorgesteuert - bremst.

In jedem Fall kann erfindungsgemäss auch bei dem Aufbau nach Fig.5 ein Sicherungsmantel 43 gemäss Fig.4b vorgesehen sein.

Beim Aufbau nach Fig.6 ist eine Keilbremse 45 zwischen den beiden Rollen gehäusefest montiert und kommt im Sicherungsfall dadurch zum Einsatz, dass das Sicherungsseil 324 gespannt wird und sich in die Keilnut 46 der Bremse einfrisst. Diese Keilbremse 45 ersetzt gegebenenfalls die oben beschriebenen Bremsmöglichkeiten gemäss Fig.5.

Beim Aufbau nach Fig.7 ist für das Klemmen des dort vertikal laufenden Sicherungsseils 324 das die Keilbremse 45 horizontal angeordnet. Da bei einem vertikal hängenden Sicherungsseil 324 nicht sichergestellt ist, dass es auf der Keilnut 46der Keilbremse 45 zu liegen kommt, ist bei dieser Ausführungsform vorzugsweise eine Andrückrolle 47 vorgesehen, die federbelastet das Sicherungsseil 324 in die ungebremste Lage drückt. Die Federkraft, welche die Andrückrolle 47 beaufschlagt, ist so gering, dass sie bei einem geringsten Spannungsanstieg am Sicherungsseil 324 überwunden wird und sich das Sicherungsseil 324 in die Keilnut 46 einfrisst.

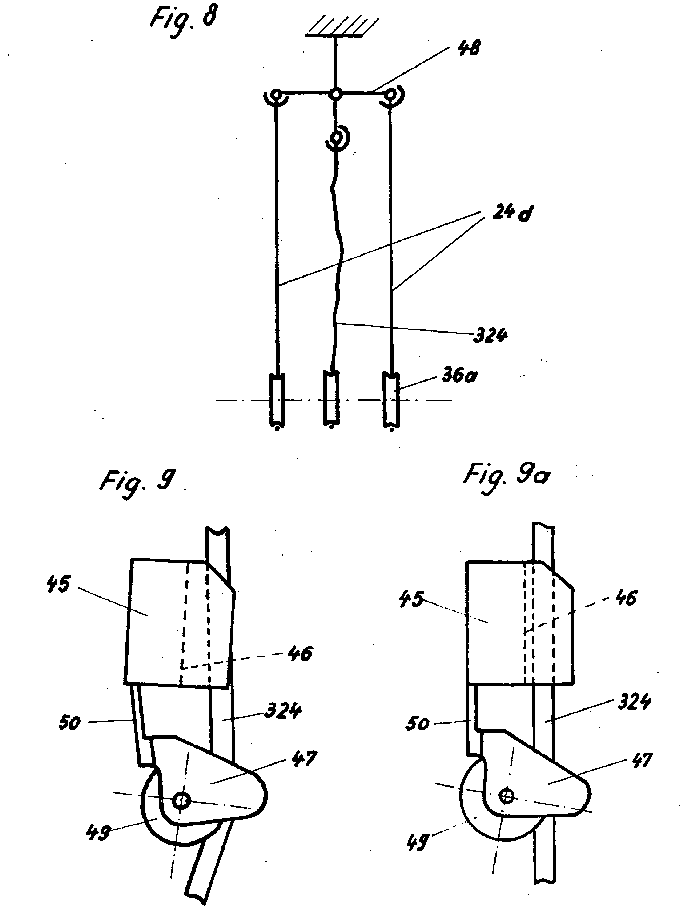

Beim Aufbau nach Fig.8 laufen zwei Arbeitsseile 24d parallel, die von einem zentralen Sicherungsseil 324 begleitet sind. Eine Wippe 48, an der die drei Seile 24d und 324 befestigt sind, ermöglicht optimalen Längenausgleich und verhindert, dass im Falle eines Bruchs eines Arbeitsseils 24d eine ungleiche Belastung an einem nicht sichtbaren Gegengewicht auftritt. Die dargestellten drei Umlenkrollen 36a könnten vorzugsweise auch einstückig ausgebildet sein.

Im Fall von nur zwei Arbeitsseilen24d ohne zusätzliches Sicherheitsseil 324 übernimmt die Wippe 48 ebenso eine Ausgleichsfunktion im Fall eines Bruchs eines der beiden Arbeitsseile 24d.

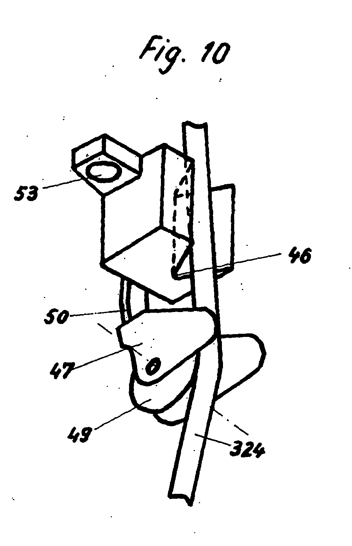

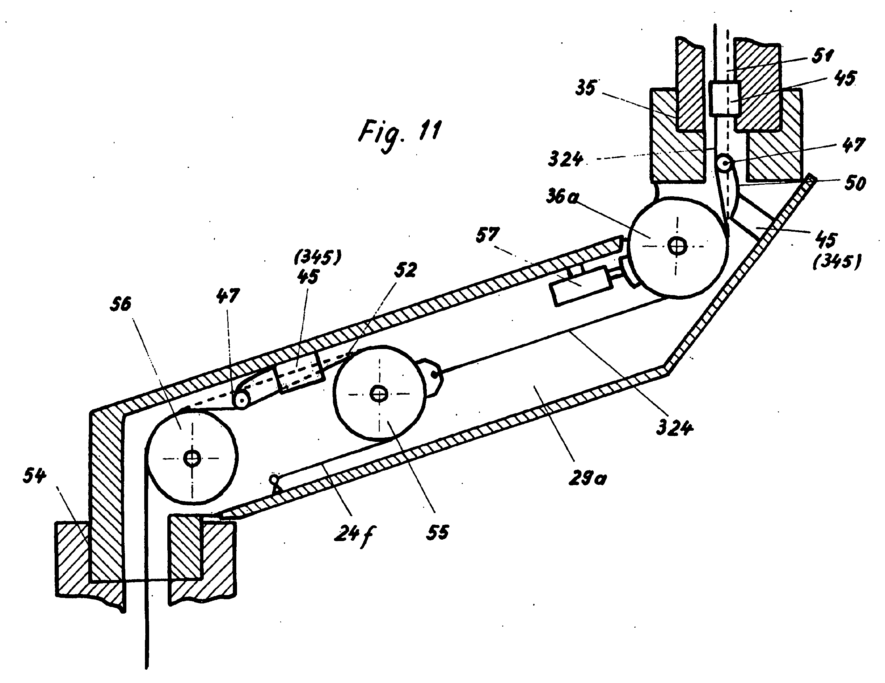

Die Fig.9 und 10 zeigen einen Sicherungsseilzug 324, der annähernd vertikal verläuft, z.b. so wie im rechten Bereich in Fig.11 oder in Fig.8. Damit sich dieser Seilzug 324 nicht unabsichtlich in die Keilbremse 45 bzw. deren Keilnut 46 frisst, ist er durch eine federbelastete Rolle 49 im Abstand von der Keilbremse 45 gehalten. Eine Blattfeder 50 erzeugt die Federkraft.

Fig. 11 zeigt einen ausgeführten Aufbau, wie er z.b. an einer Lagerstelle 54 mit einem Ständerrohr eines Stativs verbunden sein kann. Im hohlen Tragarm 29a befindet sich ein Flaschenzug 52, an dem unter anderem das Sicherungsseil 324 angreift. Der Flaschenzug 52 umfasst zwei Flaschenzugrollen 55 und 56, um welche die Seilzüge geführt sind. Wenigstens einer davon ist ein Sicherungsseilzug 24f. Auch diesen ist eine Keilbremse 45 mit Andrückrolle 47 zugeordnet, um im Fall eines Bruchs eines Seilzugs im Flaschenzug den Sicherungsseilzug 24f abzubremsen.

An einem Lager 35 ist, wie hier nicht dargestellt, z.b. der Tragarm 2 und ein Tariergetriebe schwenkbar gelagert. Zentral durch das Lager 35 verläuftt ein Hohlraum 51, welcher die Seilzüge von oben nach unten führt. Für das Sicherungsseil 324 ist die Andrückrolle 47 angeordnet, die das nicht gespannte Sicherungsseil 324 von der Keilbremse 45 fernhält. Erst im Sicherungsfall drückt das Seil 324 gegen die Rolle 47 bzw. unter Überwindung der Kraft der Feder 50 in die Keilnut 46 in der Keilbremse 45 und erschwert somit die Bedienung des Stativs.

Eine symbolisch dargestellte Bremse 57 ermöglicht das Blockieren der Umlenkrolle 36a und dadurch ein Fixieren des Stativs in seiner Lage. Gemäss einer Weiterbildung der Erfindung kann anstelle oder zusätzlich zu den dargestellten Bremseinrichtungen (z.B. Keilbremse) ein elektrischer bzw. elektronischer Endschalter 345 oder 245 vorgesehen sein, der im Sicherungsfall durch die Spannung des Sicherungsseils 324 anspricht und über Anschlusskabel 344 einen mit ihm verbundenen Stromkreis entsprechend beeinflusst. Ein solcher Stromkreis kann z.b. optische oder akustische Signale und/oder Bremsen o.dgl. ansteuern. So könnte z.b. vorgesehen sein, dass beim Einsatz von elektromagnetischen Bremsen diese nicht geöffnet werden können oder in Bremsstellung gehen.

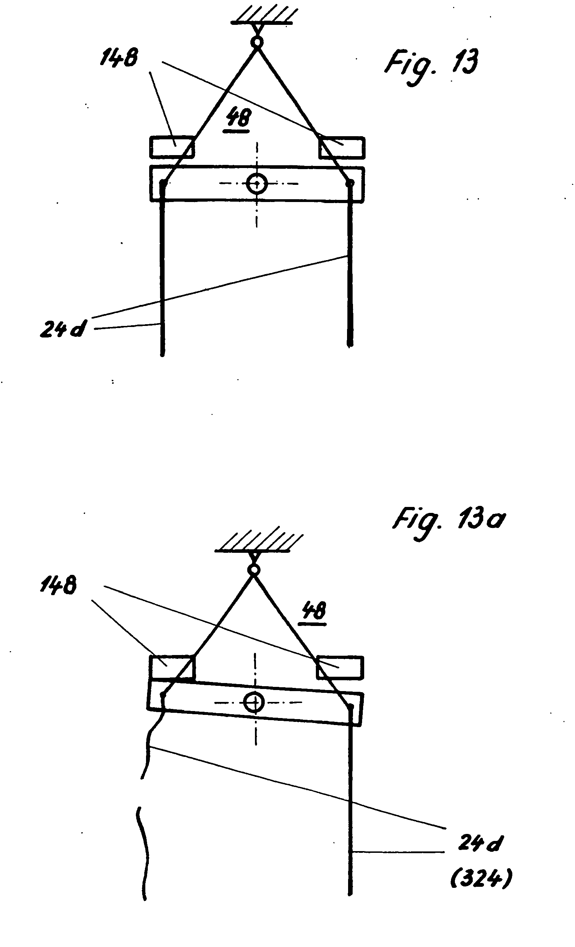

Bei der aus Fig. 13 und 13a ersichtlichen Ausführung ist eine im Sicherungsfall (d.h. Bruch eines der beiden Seile 24d) verschwenkbare Wippe 48 vorgesehen. Die Schwenkbewegung der Wippe 48 wird durch einen Anschlag 148 begrenzt, sodass das dann zum Sicherungsseil 324 gewordene Seil 24d nicht zu weit nachgeben kann. Andererseits hilft die Wippe 48, allfällige geringe Längenunterschiede auszugleichen, sodass die beiden Seile 24d im Betriebszustand gleiche Last tragen.

Im Sinne der Erfindung gibt es Lösungen, bei denen zwischen Arbeitsseilen und Sicherungsseilen nicht unterschieden werden kann, weil im Normalbetriebszustand alle Seile im Einsatz sind und unter Spannung stehen. Im Sicherheitsfall (d.h. wenn ein Seil bricht) werden die verbleibenden Seile zu je einem Sicherungsseil. Bei diesen Aufbauten sind bevorzugt alle Arbeitsseile mittels Sensoren auf Bruch überwacht, um im Sicherheitsfall entsprechende Informations- oder Bremssignale zu generieren oder auszulösen.
Bei solchen Ausführungsformen wirken die Sensoren in umgekehrter Richtung als bei reinen Sicherungsseilen. Bei letzteren detektieren sie den Aufbau einer Spannung, bei ersteren detektieren Sie den Abfall einer Spannung im Seil (Bruch).

### Bezugszeichenliste

- 2 -: Tragarm bzw. Parallelogrammträger
- 22 -: Ausgleichsarm
- 23 -: Gleitschuh
- 24 -: Seilzug
- 24d: Arbeitsseilzug
- 24f: Sicherungsseilzug
- 29a -: Tragarm
- 35 -: Lager
- 36,: 36a - Rolle
- 37,: 37a - Rolle
- 38 -: Drehbarer Teil der Umlenkrolle 37a
- 39 -: Starrer oder gebremster Teil der Umlenkrolle 37a
- 40 -: Zugfeder
- 41 -: Nut
- 42 -: Klemm- bzw. Sicherungsnut (keilförmig)
- 43 -: Sicherungsmantel
- 44 -: Achse
- 45 -: Keilbremse
- 46 -: Keilnut
- 47 -: Andrück- bzw. Sicherungsrolle
- 48 -: Wippe
- 49 -: Rollenkörper
- 50 -: Blattfeder
- 51 -: Hohlraum
- 52 -: Flaschenzug
- 55 -: Flaschenzugrollen
- 56 -: Flaschenzugrollen
- 57 -: Bremse; (vergleichbare Bremsen können auf allen Bewegungsachsen des Stativs, vorzugsweise im Tragarm 2 (Parallelogrammträger) oder im Schwenkträger 79 vorgesehen sein;)
- 245 -: Endschalter
- 324 -: Sicherungsseil
- 344 -: Anschlusskabel
- 345 -: Endschalter
- AGb -: Ausgleichsgewicht
- G -: Last bzw. Gewicht des Mikroskops
- F -: konstante Ausgleichskraft

## Patentansprüche

1. Stativ mit einem schwenkbaren Tragarm **(2)** für die Aufnahme einer Last **(G)** und mit einer Ausgleichskraft **(FA),** die über einen Arbeitsseilzug **(24)** auf den Tragarm **(2)** wirkt, **dadurch gekennzeichnet, dass** dem Seilzug **(24)** ein Sicherungsseilzug **(324)** zur Seite gestellt ist, dessen Seil **(324)** im Sicherungsfall die Funktion des Arbeitsseilzugs **(24)** übernimmt.

2. Stativ mit einem Tragarm **(2)** für die Aufnahme einer Last **(G)** und mit einer Ausgleichskraft (FA), die über einen Arbeitsseilzug (24) auf den Tragarm **(2)** wirkt, **dadurch gekennzeichnet, dass** ein Sicberungsseilzug (324) im Gebrauchszustand spannungslos parallel zum Arbeitsseitung (24), im Sicherungszustand jedoch unter Spannung verläuft.

3. Stativ nach Anspruch **1** oder **2, dadurch gekennzeichnet, dass** die beiden Seilzüge **(24, 324)** im Betriebszustand im wesentlichen parallel zueinander verlaufen und dem Sicherungsseilzug **(324)** eine Bremseinrichtung **(39; 42; 42a; 45)** zugeordnet ist, die im Fall eines Wechsels vom Arbeitsseil **(24d)** auf das Sicherungsseil **(324)** in Kraft tritt und dem Bewegen des Sicherungsseils **(324)** oder eines anderen bewegbaren Bauteils des Stativs einen Widerstand entgegensetzt, der zu einem erhöhten Widerstand gegen das Bewegen der Last **(G)** führt.

4. Stativ nach einem der vorhergehenden Ansprüche, **dadurch gekennzeichnet, dass** dem Sicherungsseil **(324)** wenigstens ein elektrischer oder elektronischer Schalter **(245; 345)** zugeordnet ist, der im Sicherungsfall durch das Sicherungsseil **(324)** direkt oder indirekt betätigbar ist und der Schalter **(245; 345)** mit Signalgebern und/oder Steuerungen und/oder elektromagnetischen Bremsen **(57)** zur Ansteuerung derselben verbunden ist.

5. Stativ nach einem der vorhergehenden Ansprüche **dadurch gekennzeichnet, dass** die beiden Seilzüge **(24, 324)** wenigstens um eine Umlenkrolle **(36a)** geführt sind, die parallele Umfangsnuten **(41, 42)** für beide Seilzüge **(24; 324)** aufweisen, wobei die Umfangsnut für das Sicherungsseil **(324)** als Klemmnut **(42)** ausgebildet ist, in die sich das Sicherungsseil **(324)** unter Zugbelastung im Sicherungsfall hineinfrisst, sodass es zu einer erhöhten Reibung zwischen Sicherungsseil **(324)** und den Wandungen der Nut **(42)** kommt.

6. Stativ nach einem der vorhergehenden Ansprüche, **dadurch gekennzeichnet, dass** der Sicherungseitzung (324) um eine Umlenkrolle **(37a)** geführt und diese zweiteilig ausgebildet, ist und über einen rotierbaren Teil **(38)** und einen ggf. gebremst rotierbaren oder starren Teil **(39)** verfügt, wobei zwischen dem rotierbaren **(38)** und dem schlecht oder nicht rotierbaren Teil **(39)** eine Klemmnut **(42a)** für den Sicherungsseilzug **(324)** derart ausgebildet ist, dass beim Auftreten einer Zugspannung am Sicherungsseilzug **(324)** sich dieses in die Klemmnut **(42a)** frisst und die beiden Teile **(38, 39)** im Sicherungsfall durch Spreizung gegen eine Federkraft auseinander verschiebbar sind, wobei dem einen Teil **(39)** eine Bremseinrichtung zur Seite gestellt ist.

7. Stativ nach Anspruch 5 oder 6, **dadurch gekennzeichnet, dass** im Normalbetrieb die Klemmnut **(42; 42a)** für das Sicherungsseil **(324)** durch einen Sicherungsmantel **(43)** abgedeckt ist, der das ungespannte Sicherungsseil **(324)** stützt, der jedoch im Falle einer erhöhten Zugspannung am Sicherungsseil **(324)** kollabiert oder bricht und das Einfressen des Sicherungsseils **(324)** in der Nut **(42; 42a)** ermöglicht.

8. Stativ nach einem der vorhergehenden Ansprüche, **dadurch gekennzeichnet, dass** ein Bremselement **(45)** vorgesehen ist, das eine Klemmnut **(46)** aufweist, die so über dem Sicherungsseil **(324)** platziert ist, dass im unbelasteten Zustand des Sicherungsseils **(324)** keine Berührung zwischen Klemmnut **(46)** und Sicherungsseil **(324)** stattfindet, dass jedoch im Falle einer am Sicherungsseil **(324)** angreifenden Zuglast das Sicherungsseil **(324)** in Klemmkontakt mit der Klemmnut **(46)** kommt.

9. Stativ nach einem der vorhergehenden Ansprüche, **dadurch gekennzeichnet, dass** wenigstens zwei parallele Arbeitsseile **(24d)** und mindestens ein dazu parallel geführtes Sicherungsseil **(324)** vorgesehen sind, wobei zum optimalen Längenausgleich zwischen den mindestens drei Seilen **(24d; 324)** eine Wippe **(48)** vorgesehen ist, welche die Anfangsenden der Arbeits- und Sicherungsseile **(24, 324)** aufnimmt.

10. Stativ nach einem der vorhergehenden Ansprüche, **dadurch gekennzeichnet, dass** zur Aufnahme der Ausgleichskräfte wenigstens zwei Arbeitsseile **(24d)** vorgesehen sind, von denen jedes für die volle Ausgleichskraft ausgelegt ist, und dass jedem dieser Arbeitsseile **(24d)** wenigstens je ein Sensor **(245; 345)** zugeordnet ist, der den Übergang vom Arbeitszustand in den Sicherungszustand registriert und Informationen an den Anwender weiterleitet und/oder Bremsen steuert.

## Claims

1. Stand having a pivotable support arm (2) to accommodate a load (G) and having a balancing force (FA) which acts on the support arm (2) via a working cable pull (24), **characterized in that** a safety cable pull (324) is placed to the side of the cable pull (24), its cable (324) taking over the function of the working cable pull (24) in the safety case.

2. Stand having a support arm (2) to accommodate a load (G) and having a balancing force (FA) which acts on the support arm (2) via a working cable pull (24), **characterized in that** during use, a safety cable pull (324) is untensioned parallel to the working cable pull (24), but in the safety state runs under tension.

3. Stand according to Claim 1 or 2, **characterized in that** the two cable pulls (24, 324) run substantially parallel to each other in the operating state and the safety cable pull (324) is assigned a braking device (39; 42; 42a; 45) which, in the event of a change from the working cable (24d) to the safety cable (324), comes into force and opposes the movement of the safety cable (324) or of another movable component of the stand with a resistance which leads to an increased resistance to the movement of the load (G).

4. Stand according to one of the preceding claims, **characterized in that** the safety cable (324) is assigned at least one electrical or electronic switch (245; 345) which, in the safety case, can be operated directly or indirectly by the safety cable (324) and the switch (245; 345) is connected to signal generators and/or controllers and/or electromagnetic brakes (57) in order to activate the same.

5. Stand according to one of the preceding claims, **characterized in that** the two cable pulls (24, 324) are led at least around a deflecting roller (36a) which has parallel circumferential grooves (41, 42) for both cable pulls (24; 324), the circumferential groove for the safety cable (324) being designed as a clamping groove (42), into which the safety cable (324) bites under tensile loading in the safety case, so that increased friction occurs between the safety cable (324) and the walls of the groove (42).

6. Stand according to one of the preceding claims, **characterized in that** the safety cable pull (324) is guided around a deflecting roller (37a) and the latter is designed in two parts and has a rotatable part (38) and a part (39) which may possibly be rotatable with braking or rigid, a clamping groove (42a) for the safety cable pull (324) being formed between the rotatable (38) and the poorly or non-rotatable part (39), such that when a tensile stress occurs in the safety cable pull (324), the latter bites into the clamping groove (42a) and the two parts (38, 39) can be displaced away from each other in the safety case by being spread counter to a spring force, the one part (39) being given a braking device to the side.

7. Stand according to Claim 5 or 6, **characterized in that** in normal operation, the clamping groove (42; 42a) for the safety cable (324) is covered by a safety jacket (43), which supports the untensioned safety cable (324) but, in the case of an increased tension on the safety cable (324), collapses or breaks and permits the safety cable (324) to bite into the groove (42; 42a).

8. Stand according to one of the preceding claims, **characterized in that** a braking element (45) is provided which has a clamping groove (46), which is placed above the safety cable (324) in such a way that, in the unloaded state of the safety cable (324), no contact takes place between the clamping groove (46) and safety cable (324), but in that, in the case of a tensile load acting on the safety cable (324), the safety cable (324) comes into clamping contact with the clamping groove (46).

9. Stand according to one of the preceding claims, **characterized in that** at least two parallel working cables (24d) and at least one safety cable (324) guided in parallel therewith are provided, for optimum length balancing between the at least three cables (24d; 324), a rocker (48) being provided, which accommodates the starting ends of the working and safety cables (24, 324).

10. Stand according to one of the preceding claims, **characterized in that** in order to accommodate the balancing forces, at least two working cables (24d) are provided, each of which is designed for the full balancing force, and **in that** each of these working cables (24d) is assigned at least one sensor (245; 345) each, which registers the change from the working state to the safety state and passes on information to the user and/or controls brakes.

## Revendications

1. Support avec un bras support pivotant (2) destiné à réceptionner une charge (G) et avec une force équilibrante (FA), qui par l'intermédiaire d'un tirant à câble de service (24) agit sur le bras support (2), **caractérisé en ce qu'**un tirant à câble de sécurité (324) dont le câble (324) assure la fonction du tirant à câble de service (24) en position de sécurisation est associé au tirant à câble (24).

2. Support avec un bras support (2) destiné à réceptionner une charge (G) et avec une force équilibrante (FA), qui par l'intermédiaire d'un tirant à câble de service (24) agit sur le bras support (2), **caractérisé en ce qu'**en position de service, un tirant à câble de sécurité (324) s'étend sans tension, à la parallèle du tirant à câble de service (24) mais qu'en position de sécurisation, il s'étend sous tension.

3. Support selon la revendication 1 ou 2, **caractérisé en ce qu'**en position de service, les deux tirants à câble (24, 324) s'étendent essentiellement à la parallèle l'un de l'autre et **en ce qu'**un dispositif de freinage (39 ; 42 ; 42a ; 45), qui entre en service dans le cas d'un changement du câble de service (24d) au câble de sécurité (324) et qui oppose au déplacement du câble de sécurité (324) ou d'un autre élément mobile du support une résistance qui a pour effet de générer une résistance accrue au déplacement de la charge (G), est associé au tirant à câble de sécurité (324).

4. Support selon l'une quelconque des revendications précédentes, **caractérisé en ce qu'**au moins un interrupteur électrique ou électronique (245 ; 345), qui en position de sécurisation est actionnable directement ou indirectement par le câble de sécurité (324) est associé au câble de sécurité (324) et **en ce que** l'interrupteur (245 ; 345) est relié à des transmetteurs de signaux et/ou à des systèmes de commande et/ou à des freins électromagnétiques (57) pour activer ces derniers.

5. Support selon l'une quelconque des revendications précédentes, **caractérisé en ce que** les deux tirants à câble (24, 324) sont guidés au moins autour d'une poulie de renvoi (36a), qui comporte des rainures périphériques parallèles (41, 42) pour les deux tirants à câble (24 ; 324), la rainure périphérique pour le câble de sécurité (324) étant conçue en tant que rainure de serrage (42) dans laquelle le câble de sécurité (324) s'engage sous traction en position de sécurisation, pour donner naissance à un frottement accru entre la câble de sécurité (324) et les parois de la rainure (42).

6. Support selon l'une quelconque des revendications précédentes, **caractérisé en ce que** le tirant à câble de sécurité (324) est guidé autour d'une poulie de renvoi (37a) et **en ce que** celle-ci est conçue en deux parties et dispose d'une partie rotative (38) et d'une partie rotative, freinée le cas échéant ou rigide (39), une rainure de serrage (42a) pour le tirant à câble de sécurité (324) étant conçue entre la partie rotative (38) et la partie difficilement ou non rotative (39) de façon telle, que lors de l'exercice d'un effort de traction sur le tirant à câble de sécurité (324), ce dernier s'engage dans la rainure de serrage (42a) et en position de sécurisation, les deux parties (38, 39) soient déplaçables de façon à s'éloigner l'une de l'autre par écartement contre une tension du ressort, un dispositif de freinage étant adjoint à l'une des parties (39).

7. Support selon la revendication 5 ou 6, **caractérisé en ce qu'**en fonctionnement normal, la rainure de serrage (42 ; 42a) pour le câble de sécurité (324) est recouverte par une enveloppe de protection (43) qui supporte le câble de sécurité (324) non tendu, mais qui dans le cas d'un effort de traction accru sur le câble de sécurité (324) s'effondre ou se rompt et permet l'engagement du câble de sécurité (324) dans la rainure (42 ; 42a).

8. Support selon l'une quelconque des revendications précédentes, **caractérisé en ce qu'**on a prévu un élément de freinage (45) qui comporte une rainure de serrage (46) qui est placée au-dessus du câble de sécurité (324), de façon à ce que lorsque le câble de sécurité (324) n'est soumis à aucune contrainte, il n'y ait aucun contact entre la rainure de serrage (46) et le câble de sécurité (324), mais que dans le cas d'une contrainte en traction agissant sur le câble de sécurité (324), le câble de sécurité (324) entre en contact de serrage avec la rainure de serrage (46).

9. Support selon l'une quelconque des revendications précédentes, **caractérisé en ce qu'**on a prévu au moins deux câbles de service parallèles (24d) et au moins un câble de sécurité (324) guidé à la parallèle par rapport à ces derniers, un archet (48) réceptionnant les extrémités antérieures des câbles de service et de sécurité (24, 324) étant prévu pour une compensation de longueur optimale entre les au moins trois câbles (24d ; 324).

10. Support selon l'une quelconque des revendications précédentes, **caractérisé en ce que** pour l'absorption des forces équilibrantes, on a prévu au moins deux câbles de service (24d) dont chacun est conçu pour la pleine force équilibrante et **en ce que** respectivement au moins un capteur (245 ; 345) qui enregistre le passage de l'état de service à l'état de sécurisation et qui transmet à l'utilisateur des informations et/ou qui commande les freins est associé à chacun de ces câbles de service (24d).
